(19) Europäisches Patentamt
European Patent Office
Office européen des brevets

(11) Publication number: 0 074 191
B1

(12) **EUROPEAN PATENT SPECIFICATION**

(45) Date of publication of patent specification: **12.03.86**

(21) Application number: **82304293.2**

(22) Date of filing: **13.08.82**

(51) Int. Cl.$^4$: **A 61 K 7/11,** C 08 F 226/06 // (C08F226/06, 226:10, 246:00)

(54) **Hair treatment preparation containing vinyl caprolactam/vinyl pyrrolidone/alkylacrylate polymer.**

(30) Priority: **13.08.81 US 292329**

(43) Date of publication of application:
**16.03.83 Bulletin 83/11**

(45) Publication of the grant of the patent:
**12.03.86 Bulletin 86/11**

(84) Designated Contracting States:
**AT BE CH DE FR GB IT LI NL SE**

(56) References cited:
**FR-A-1 446 550**
**FR-A-2 229 391**
**FR-A-2 393 573**
**US-A-3 145 147**

(73) Proprietor: **GAF CORPORATION**
**140 West 51st Street**
**New York New York 10020 (US)**

(72) Inventor: **Lorenz, Donald H.**
**12 Radel Place**
**Basking Ridge, N.J. 07920 (US)**
Inventor: **Murphy, Edward J.**
**38 Clinton Lane**
**Wayne, N.J. 07470 (US)**
Inventor: **Rutherford, John M.**
**46 Cabot Lane**
**Kinnelon, N.J. 07405 (US)**

(74) Representative: **Ford, Michael Frederick et al**
**MEWBURN ELLIS & CO. 2/3 Cursitor Street**
**London EC4A 1BQ (GB)**

**EP 0 074 191 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to terpolymers and to cosmetic preparations (especially hair setting and conditioning compositions) containing them.

In the field of hair care, setting, waving, conditioning and the like, several broad types of hair treating preparations have been proposed, the principal ones being cationic surfactants, superfatting materials, water soluble proteins and synthetic polymers, in a suitable cosmetically acceptable medium. The synthetic polymer-containing preparations are generally regarded as most effective, particularly those containing water soluble cationic polymers which are substantive to hair and exhaust thereon from solution or diluent medium. British Patent No. 1,331,819 and U.S. Patent Nos. 3,910,862, 3,914,403 and 3,954,960, the disclosures of which are incorporated herein for reasons which will become apparent, describe water soluble cationic quaternized copolymers of vinyl pyrrolidone (N-vinyl-2-pyrrolidone), hereafter referred to as VP and a dialkylaminoalkyl acrylate or methacrylate, which have been found to be highly effective in providing most of the properties considered necessary in the theoretically perfect hair preparation, as in fact also described in said patents. The hair preparations described in said patents are however not optimal in certain respects, notably a high cost of producing the quaternized copolymers, a curl retention which is not as good as could be desired under high humidity conditions, and as an ease of removability and/or resistance to build-up which is also not as high as could be desired.

U.S. Patent No. 4,223,009 attempts to provide an improved system through the use of a copolymer of 99.5 to 45.1 mole percent vinyl pyrrolidone, 0.5 to 4.9% of an acrylate and 0 to 50% of an ethylenically unsaturated copolymerizable monomer such as the alkyl vinyl ethers, e.g. the methyl, ethyl, octyl and lauryl vinyl ethers; acrylic and methacrylic acid and esters thereof, e.g. methyl acrylate, ethyl acrylate and methyl methacrylate; vinyl aromatic monomers, e.g. styrene and alpha-methyl styrene; vinyl acetate and chloride; vinylidene chloride; acrylonitrile and methacrylonitrile and substituted derivatives thereof; acrylamide and methacrylamide and N-substituted derivatives thereof; crotonic acid and esters thereof, e.g. methyl and ethyl crotonate; and the like.

The patent broadly defines the acrylate as being a monomer of the formula

$$CH_2{=}CR^1{-}COOR^2{-}NR^3R^4$$

wherein

$R^1$ is H or $CH_3$,

$R^2$ is $C_{1-20}$ alkylene, and

$R^3$ and $R^4$ are independently $C_{1-4}$ alkyl.

U.S. Patent No. 4,223,009 further indicates that it was highly surprising to discover that elimination of the quaternization step required in accordance with the teachings of said patents not only did not result in any significant detriment to the properties of the hair preparations containing such polymers, but proved advantageous in substantially reducing the costs of manufacture and in providing hair preparations yielding improved properties in the treated hair with respect of improved curl retention under high humidity conditions, ease of removability and/or resistance to build-up with repeated use, among other miscellaneous advantages.

The shortcomings of the use of poly-N-vinyl-2-pyrrolidone (PVP) in hair conditioning formulations is expounded upon in U.S. Patent No. 3,145,147. According to the patent current popular aerosol hair spray composition contains as the hair setting and waving medium, poly-N-vinyl-pyrrolidone (PVP). When a PVP-alcohol solution is applied in an aerosol system to human hair under a relative humidity of less than 50%, the tendency of the resulting film to tackiness is minimal. As a result thereof, the preparation is acceptable by all consumers. However, when the relative humidity is above 50%, and particularly in humid atmospheres, films of PVP obtained by spraying from an aerosol system, pick up considerable moisture. The moisture is retained and results in a tacky film. In view of this property of moisture retention, these aerosol preparations are extremely undesirable where a dry hair condition is required as is the case with most users, especially women. The equilibrium water content of PVP depends upon the relative humidity of the atmosphere. The moisture content varies in a linear fashion with relative humidity, and the equilibrium percentage of moisture is about one-third of the relative humidity. Thus if PVP is exposed to a relative humidity of 50%, the moisture pick-up is approximately one-third of the relative humidity, and therefore the resulting film contain about 13—14% moisture. To overcome the unique hygroscopicity of PVP, it has been suggested in the cosmetic art to employ detackifying agents such as shellac, cellulose acetate-propionate, etc. The former yields films which become opaque at high humidities, and the latter yields films which are insoluble to ethyl alcohol. Carboxymethyl cellulose, cellulose acetate, methyl methacrylate polymer, polyvinyl formal, etc. are not effective as detackifiers under conditions of extremely high humidities.

Another drawback of PVP is that in the course of its manufacture and handling, the polymer picks up sufficient moisture (water from the atmosphere) to substantially modify its solubility in solvents, other than lower alcohols, such as acetone, methylene chloride, etc. and prohibits its use in formulation of aerosol propellent mixtures. However, storage of PVP at 50% relative humidity yields a material which possesses solubility at a 10% level in absolute ethanol but is not completely soluble in acetone and methylene

chloride. One prime reason which rules out the use of PVP in the formulation of aerosol compositions for the application of films to surfaces other than hair is its insolubility or rather insufficient solubility in acetone and methylene chloride. In the formulation of protective film coatings for surfaces such as silver and silver plate ware to protect them from tarnishing, etc., coupled with fast drying, a concentration of the film forming medium must be at least 5% and usually the preferred concentration being 10% and higher.

In order to provide a formulation which does not have the shortcoming of PVP systems, the US 3,145,147 patent advocates the use of copolymers of N-vinyl-ε-caprolactam. The patent discloses that the copolymerizable compounds which may be copolymerized with N-vinyl-ε-caprolactam include vinyl esters such as vinyl acetate, vinyl isopropenyl acetate and the like; alkyl acrylates such as methyl acrylate, ethyl acrylate, methyl methacrylate and the like; acrylamides such as acrylamide, methacryamide and the like; acrylonitriles such as acrylonitrile, methacrylonitrile and the like; alkyl vinyl ethers such as methyl vinyl ether, ethyl vinyl ether, isopropenyl methyl vinyl ether and the like.

Generally, the above mentioned U.S. patent demonstrates the greater water sensitivity of vinyl pyrrolidone polymers over that of vinyl caprolactam polymers.

The present invention provides a hair conditioning composition consisting of or containing a terpolymer derived from vinyl pyrrolidone monomer, an ammonium derivative monomer and a substantially major amount based on said monomers of vinyl caprolactam. The polymeric composition may optionally contain a minor amount of stearyl methacrylate. Suitable ammonium derivative monomers include dimethylamino propyl methacrylamide, dimethyl diallyl ammonium chloride and a dialkylamino alkyl methacrylate, such as dimethylamino ethyl methacrylate. It has been found that copolymer made from the above monomers can be used to produce surprisingly high quality, low cost hair conditioning products.

Preparation of the terpolymer from the three monomers can be performed by conventional techniques used for polymerising these monomers.

We have found that polyvinyl caprolactam and polyvinyl pyrrolidone display a surprising synergistic behaviour in withstanding high humidity holding failure. This has been established by testing hair samples treated with instant terpolymer at 90% relative humidity at 80°F (27°C).

Polyvinyl pyrrolidone is known to be a very hydroscopic resin; whereas polyvinyl caprolactam is much less so. The hydroscopicity of the former resin causes failure to retain hair curl under high humidity conditions. The polyvinyl caprolactam, being substantially less hydroscopic retains hair curl under these conditions. Thus, it was completely unexpected, that partial replacement of vinyl caprolactam (VCPL) by vinyl pyrrolidone (VP) results in an improvement in high humidity holding. In fact, this effect increases as the VP was increased. However, the amount of VP incorporation is limited by the tackiness of the resin which occurred when significantly more than 25% by weight of the final polymer was vinylpyrrolidone.

Another critical parameter is dependent upon resin build-up on the hair. In order to effect removal of the copolymer during shampooing usually at about 100°F (38°C)—it is very desirable to change the characteristics of polyvinyl caprolactam which becomes insoluble in water above 30°C. (86°F.). Unexpectedly, the incorporation of dimethylaminoethyl methacrylate did not change the cloud-clear point (temperature at which the resin loses solubility). However, replacement of vinyl caprolactam by vinyl pyrrolidone does afford a significant change in cloud-clear point, and hence an improvement in shampooability.

Comparative trials were undertaken, as follows.

Poly vinyl caprolactam (PVCPL) homopolymer was compared with polyvinyl pyrrolidone (PVP), poly vinyl pyrrolidone/vinyl acetate (VP/VA), VP/VA Itaconic Acid and a commercially available material sold by GAF Corporation under the registered trademark GANTREZ, and designated as GANTREZ ES225 which is the ethyl monoester of poly (methyl vinyl ether/maleic acid) of 50% solids in ethanol. The formulations were used in anhydrous type hydrocarbon propelled hair spray formulations and compared for spray pattern, compatibility, hair characteristics and humidity holding. The PVCPL was found to be comparable to GANTREZ ES255, which is generally recognized as being a superior quality product. The PVCPL was superior to the other 5 resins tested.

Four vinyl caprolactam (VCPL) copolymers were compared with a PVCPL control, with the following results:

a) VCPL/Stearyl Methacrylate (97/3)—Hair Tress humidity holding was significantly less than the control. On hair tress characteristics this polymer showed a little better ease of combing compared to the control.

b) VCPL/Methyl Methacrylate (95/5)—This polymer was inferior in hair tress humidity holding and showed no advantages in hair tress characteristics when compared to the control.

c) VCPL/Dimethylamino ethyl methacrylate (95/5) quaternized with Propylene Oxide (PO)—This polymer was significantly superior to the control in hair tress humidity holding. In hair tress characteristics, it gave very difficult combing attributes. Since this polymer is cationic it was further evaluated in a hydroalcoholic hydrocarbon system. Whereas the concentrate was clear, the charged unit was hazy. A hair tress humidity hold showed it held significantly better than the control. It was not compared to the anhydrous VCPL/Dimethylamino ethyl methacrylate system. A pyrazol red test on albino hair gave a light pink indicating some cationic material is retained on the hair.

d) VCPL/Acrylic Acid—This polymer showed slight hazing in the concentrate and definite clouding in

the charged unit when formulated unneutralized. Evaluation of 25%, 50%, 75% and 100% stoichiometric neutralizations showed the 100% gave a clear finished product. This formulation was evaluated and was inferior in hair tress humidity holding and offered no advantages in hair tress characteirstics.

The outstanding result from these evaluations is the substantial increase in holding with only 5% dimethylamino ethyl methacrylate (DMAEMA)+propylene oxide (PO). In addition, the small amount of stearyl methacrylate (SMA) did appear to be helping the ease of combing, as would be expected. It was also noted that the compability of the quaternized DMAEMA copolymer was decreased.

Several additional sets of resins were then prepared and evaluated in anhydrous alcoholic systems, with the following compositional variables being explored:

a) Low levels of long chain alkyl groups for expected improved compatability (quaternization of the DMAEMA with a C—16 epoxide or glycidyl dodecyl ether, as 92/5/3 VCPL/DMAEMA+PO/SMA).

b) DMAEMA level.

c) Effect of not quarternizing.

d) Replacing some of the VCPL with VP.

A group of eight resins was selected for evaluation in both anhydrous and hydroalcoholic aerosols. Compatibility data for these resins are shown in Table I, where it is apparent that replacement of a small amount of VCPL with SMA gives a significant improvement. VCPL/SMA/DMAEMA+PO (92/3/5) as the only resin whose concentrate, anhydrous and hydroalcoholic aerosols were all clear, and it gave the best cloud/clear point in the hydroalcoholic system.

The hair tress humidity hold studies indicated the following:

a) Quartenization is of no value in either system.

b) DMAEMA improves holding in both systems, and in the hydroalcoholic system. The DMAEMA level (2.5 to 10%) correlated well with the degree of holding.

c) All resins hold better in the hydroalcoholic system.

With regard to hair tress characteristics, all the test resins gave less comb residue than GANTREZ ES—225 but gave more comb drag and left the hair feeling more coated and tacky, although a preliminary study with cast films indicated two of the test resins as being less tacky than GANTREZ ES—225.

TABLE I
Compatibility of selected vinyl caprolactam polymers in aerosol hairspray systems

| Polymer composition | Super concentrate[a] | Alcoholic aerosol | | Hydroalcoholic aerosol | |
|---|---|---|---|---|---|
| | | Visual | Cloud/clear point[b] | Visual | Cloud/clear point[b] |
| VCPL | Hazy | Sl. Hazy | <−20°F | Clear | 18°F/26°F |
| VCPL/DMAEMA (95/5) | V. Sl. Hazy | V. Sl. Hazy | <−20°F | Clear | 16°F/20°F |
| VCPL/DMAEMA+PO (97.5/2.5) | Clear | Hazy | — | Hazy | — |
| VCPL/DMAEMA+PO (95/5) | Clear | Hazy | — | Very cloudy | — |
| VCPL/DMAEMA+PO (90/10) | Very cloudy | Cloudy, ppt. | — | Cloudy, ppt. | — |
| VCPL/VP/DMAEMA+PO (71/24/5) | Clear | Very hazy | — | Cloudy | — |
| VCPL/SMA/DMAEMA+PO (92/3/5) | Clear | Clear | <−20°F | Clear | 10°F/12°F |
| VCPL/VP/DMAEMA+C—16 Epoxide (71/24/5) | V. Sl. Hazy | Hazy | — | Hazy | — |

[a]3.75% solids in anhydrous ethanol. Concentrate is filtered before dilution to hydroalcoholic (8% water) or alcoholic aerosols (25% hydrocarbon propellent).
[b]Cloud/clear points were not on hazy products.

TABLE II
Cloud temperatures of vinyl caprolactam copolymers

| Copolymer composition, mole% | | | |
|---|---|---|---|
| VP | VCPL | DMAEMA+PO | Cloud temperature[a] |
| 0 | 100 | 0 | 32.3°C |
| 29.5 | 70.5 | 0 | 43.5 |
| 55.6 | 44.4 | 0 | 61.5 |
| 79 | 21 | 0 | >100 |
| 0 | 95.5 | 4.5 | 32.5 |
| 27.9 | 67.6 | 4.5 | 43.4 |
| 52.9 | 42.6 | 4.5 | 62.4 |
| 75.3 | 20.2 | 4.5 | >100 |
| 85.6 | 9.9 | 4.5 | >100 |

[a]Determined by diluting a 40% solution in ethanol with water to 5% and slowly heating with stirring on a hot plate until the polymer precipitated.

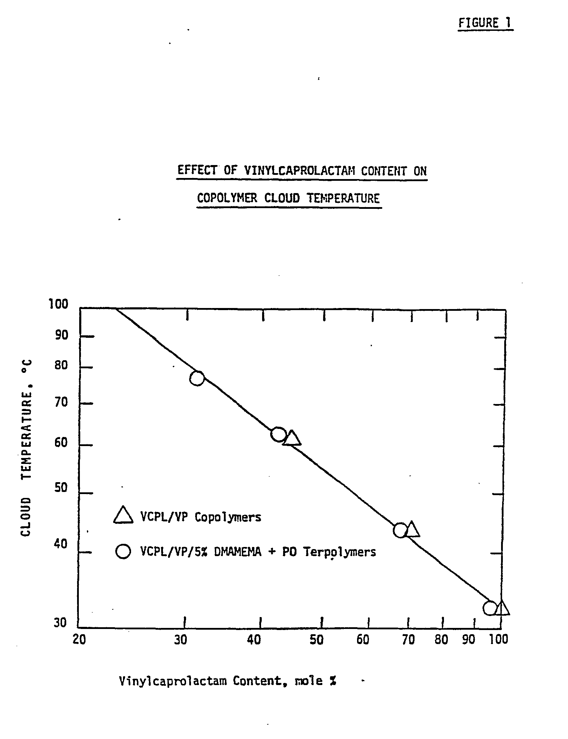

Series of VCPL/VP copolymers and VCPL/VP/5% DMAEMA+PO terpolymers were prepared for determination of their cloud temperatures (i.e., the temperature above which they precipitate from aqueous solution), with the thought that increasing the hydrophilicity of the resin should improve its shampooability. These data are shown in Table II, and when the cloud temperatures are plotted against VCPL content of the copolymers, they fall on a common line showing an inverse correlation in a log-log plot with an extrapolated 100°C cloud temperature at about 23 mole per cent VCPL, as shown in Figure 1. It can be seen that incorporation of DMAEMA into VCPL did not increase the cloud temperature to any appreciable extent, but incorporation of VP did do so.

All of the resins under evaluation had a 2.5% solids content which is the optimum level for the ANTREZ ES—225 resin. The high comb drag and "coated" feel of the hair observed with the VCPL resins was determined to attribute to the unnecessarily high level of VCPL and could be avoided through the use of decreased VCPL. Surprisingly, the VCPL exhibits a substantially better humidity holding power than GANTREZ ES—225 on an equal weight basis, and therefore, can be used at a decreased weight level without adversely affecting its humidity holding power.

Two series of unquarternized resins were evaluated at a 1.5% solids concentration level. In one series, VP levels were varied from 48 to 95% for improved shampooability and in the other series, VP and VCPL contents were equal, but DMAEMA levels were varied from 1% to 10%. The results were as follows:

a) All VCPL resins at 1.5% were equal to or better than GANTREZ ES—225 at 2.5% on humidity holding.

b) All left less residue on the hair and comb than GANTREZ ES—225.

c) They all shampooed well, indicating that earlier observed shampooability problems were an artifact of overdosing and/or hair preparation technique.

d) Higher VP levels improved humidity holding.

e) Higher VP levels increased the tack.

To test hair curl humidity holding, hair tresses were treated with the various polymers at the 1.5% solids level in hydroalcoholic and anhydrous alcoholic systems. They were dried under ambient conditions and the height of curl above a reference point measured. The tresses were exposed to 90% relative humidity (R. H.) at 80°F (27°C) and the height of curl measured again after the elapse of various periods. The height which remains is expressed as a percentage of the original height.

Some significant results, showing the percentage curl retention after 45 minutes are shown in Table III.

TABLE III

| Polymer composition VP/VCPL/DMAEMA | VP/VCPL ratio | % curl retention* hydroalcoholic system | anhydrous alcoholic system |
|---|---|---|---|
| 0/95/5 | 0/100 | 63 | 64 |
| 19/76/5 | 2080 | 78 | 81 |
| 47.5/47.5/5 | 50/50 | 89 | 90 |

*% curl retention measured after 45 minutes at 90% relative humidity, 80°F (27°C).

It was entirely contrary to expectation that going from 95/5 VCPL/DMAEMA polymer to 47.5/47.5/5 VP/VCPL/DMAEMA terpolymer would improve the resistance to high humidity holding fade.

A tack test was designed where the product is sprayed on a glass plate, dried thoroughly, and then the plate is conditioned under constant temperature and humidity. Three cotton balls are gently laid on the film. At 20, 40 and 60 minutes, one of the balls is finger pressed onto the film and then removed. The amount of cotton left on the film is indication of tackiness. The butyl monoester of poly (methyl vinyl ether/maleic anhydride) of 50% solids in ethanol (GANTREZ ES—425) and the ethyl monoester of poly (methyl vinyl ether/maleic acid) of 50% solids in ethanol (GANTREZ ES—225) as well as PVP K—30, were used as controls. All of the above N-vinyl caprolactam polymers showed tackiness quite similar to PVP K—30. However, by increasing the VCPL content to 71%, the system was equivalent to GANTREZ ES—425.

Two samples were evaluated for tackiness properties according to Materials Research and Testing No. 82/01:

"Tackiness Test for Solvent Based Films".

Films of each resin were cast on a glass plate, dried and placed in environmental chamber. The humidity therein was gradually increased to the point when bead travel distance was interrupted due to tackiness of the film.

A sample of VP/VPCL/DMAEMA terpolymer, in proportions 47.5/47.5/5.0, and having a K value of 46.1 became tacky at 96% R. H. and 80°F (27°C). The bead travel averaged 86 mm.

A sample in which the VP/VPCL/DMAEMA proportions were 21/74/5, having a K value of 40.8 remained dry. The shot bead rolled the entire length of the glass plate.

Touching the films with a finger also indicated the increased amount of tack of the first sample when compared with the non-tacky second sample.

Another set of three resins, in which some of the VCPL was replaced with lauryl methacrylate (5%), stearyl methacrylate (5%), and vinyl acetate (10%), was prepared to determine the possible beneficial effect of including a more hydrophobic monomer but no substantial differences were noted.

The foregoing testing showed that VCPL/VP/DMAEMA in a 71/24/5 ratio gave optimum results and was equivalent to about twice the concentration of a GANTREZ resin.

DMAEMA is known to be a more reactive monomer than VP and therefore, the use of a monomer feed reaction would be expected to yield a more uniform copolymer than obtainable in a batch reactor.

The only differences noted were in the 71/24/5 composition, where the monomer feed gave resin with somewhat inferior humidity holding—one slightly so in the anhydrous system but more significantly in the hydroalcoholic one. The effect of molecular weight was also examined for a threshold molecular weight for optimum holding. Evaluation of these tests showed a high molecular weight to improve holding in the anhydrous system.

The foregoing tests showed a polymer produced from 71/24/5 ratio of VCPL/VP/DMAEMA monomers to produce a hairspray resin at least equal in quality to the best resins in commercial use, such as GANTREZ 425 and 225, while requiring approximately half the concentration level of the commercial systems.

The low cloud temperature of the 71/24/5 VCPL/VP/DMAEMA resin would be expected to render the resin unsuitable for use in hair conditioning resins for shampoo.

However, in a creme rinse formulation and an anionic shampoo, the cloud point of the same resin was found to be 50° and 70°C respectively. The creme rinse formulation is as follows:

| | |
|---|---|
| Triton X400® (stearyl dimethyl benzyl ammonium chloride) | 7.0 |
| Glyçeryl monostearate | 2.0 |
| Cerophyl 28® (cetyl lactate) | 1.0 |
| Glutaraldehyde (25%) | .4 |
| Sodium hydroxide (10%) | .26 |
| VCPL/VP/DMAEMA (38.12% solids) | 1.05 |
| $H_2O$ (distilled) s to 100 | |

An Amphoteric shampoo formulation is as follows:

| | % |
|---|---|
| Gafac RS—610® (free acid of a complexed phosphonate ester) | 6.00 |
| Miranol® cm conc. (N.P.) (dicarboxylic coconut derivative of an imidazoline, sodium salt) | 25.00 |
| Distilled water | 58.03 |
| 10% citric acid solu. | 7.00 |
| PEG 6000 distearate (polyethylene glycol of MW 6000) | 1.00 |
| Sodium sulfite | .10 |
| perfume | .25 |
| VCPL/VP/DMAEMA | 2.62 |

The anionic shampoo formulation is as follows:

| | |
|---|---|
| Distilled $H_2O$ | 53.58 |
| Sipon LT.6® (TEALS) (triethanolamine lauryl sulfate) | 35.00 |
| Monoamid CMA® (coconut monoethanolamide) | 3.00 |
| Coconut fatty acid | 2.00 |
| Triethanolamine | 1.40 |
| PEG 6000 distearate (polyethylene glycol of MW 6000) | 1.40 |
| NaCl | .60 |
| Sodium sulfite | .10 |
| perfume | .30 |
| VCPL/VP/DMAEMA | 2.62 |

A clear creme rinse formulation is as follows:

| | |
|---|---|
| Distilled water | 95.08 |
| Natrosol 250 HHR® (hydroxyethyl cellulose) | .40 |
| Ammonyx KP® (oleyl dimethyl benzyl ammonium chloride) | 4.00 |
| VCPL/VP/DMAEMA | .52 |

The foregoing formulations were comparable to those using a resin sold under the registered trademark GAFQUAT and designated as GAFQUAT 755$^N$ (the polymer of VP/DMAEMA of 20% solids in aqueous solution) with respect to substantivity, curl retention, build-up and build-up removal, but inferior in ease of wet combing.

The VCPL/VP/DMAEMA resin is free of carboxylic acid groups and accordingly should be less liable to corrosion problems of the type which would be encountered with a formulation of the type disclosed in U.S. Patent No. 4,164,562 when used with the hydroalcohol solvents of the patent.

The caprolactam-vinyl pyrrolidone ratio controls the water sensitivity of the product. Increased pyrrolidone concentrations increases the solubility of the caprolactam which becomes insoluble at temperatures greater than 30°C but increases the propensity toward tackiness at high humidity.

The third component is employed to provide substantiality, that is, the ability to adhere to hair, or other natural substances which are characteristically negatively charged.

Although polymer of the instant invention has, as its primary function, the contribution of humidity holding to the formulation, other properties can be improved through the use of other additives or comonomer. For example, stearyl methacrylate can be used in small quantities as a co-monomer in order to improve the combing characteristics without adversely influencing the humidity holding properties.

**Claims for the Contracting States: BE, CH, DE, FR, GB, IT, LI, NL, SE**

1. A hair conditioning composition consisting of or containing a terpolymer derived from a major proportion of vinyl caprolactam monomer and minor proportions of vinyl pyrrolidone monomer and an ammonium derivative monomer.

2. The hair conditioning composition of claim 1 wherein the ammonium derivative monomer is a dialkylamino alkyl acrylate or methacrylate.

3. The hair conditioning composition of claim 2 wherein the ammonium derivative monomer is dimethylamino ethyl methacrylate.

4. The hair conditioning composition of claim 3 wherein the proportion of dimethylamino ethyl methacrylate is 5 to 10 weight percent of the polymer.

5. The hair conditioning composition of any preceding claim wherein the proportion of vinyl caprolactam is at least 65 weight percent of the terpolymer and the proportion of vinyl pyrrolidone is not more than 25 weight percent of the terpolymer.

6. The hair conditioning composition of any preceding claim wherein the terpolymer also contains a minor amount of stearyl methacrylate.

7. The hair conditioning composition of claim 3 wherein the weight ratio of vinyl caprolactam to vinyl pyrrolidone to dimethylamino ethyl methacrylate is a ratio of about 70:25:5.

8. The hair conditioning composition of claim 7 wherein about 3% by weight of the vinyl caprolactam is replaced with stearyl methacrylate.

9. The hair conditioning composition of any preceding claim wherein the composition contains a surfactant.

**Claims for the Contracting State: AT**

1. A method of conditioning hair comprising applying to hair a composition consisting of or containing a terpolymer derived from a major proportion of vinyl caprolactam monomer and minor proportions of vinyl pyrrolidone monomer and an ammonium derivative monomer.

2. A method of claim 1, wherein the ammonium derivative monomer is a dialkylamino alkyl acrylate or methacrylate.

3. A method of claim 2, wherein the ammonium derivative monomer is dimethylamino ethyl methacrylate.

4. A method of claim 3, wherein the proportion of dimethylamino ethyl methacrylate is 5 to 10 weight percent of the polymer.

5. A method of any preceding claim wherein the proportion of vinyl caprolactam is at least 65 weight

percent of the terpolymer and the proportion of vinyl pyrrolidone is not more than 25 weight percent of the terpolymer.

6. A method of any preceding claim wherein the terpolymer also contains a minor amount of stearyl methacrylate.

7. A method of claim 3, wherein the weight ratio of vinyl caprolactam to vinyl pyrrolidone to dimethylamino ethyl methacrylate is a ratio of about 70:25:5.

8. A method of claim 7 wherein about 3% by weight of the vinyl caprolactam is replaced with stearyl methacrylate.

9. A method of any preceding claim wherein the composition contains a surfactant.

10. A hair conditioning composition containing a terpolymer as specified in any one of claims 1 to 8, and at least one other ingredient.

**Patentansprüche für die Vertragsstaaten: BE—CH/LI—FR—IT—NL—SE—DE—GB—**

1. Eine Haarkonditionierungszusammensetzung, bestehend aus oder enthaltend ein(em) Terpolymer, das aus einem größeren Anteil von Vinylcaprolactammonomer und kleineren Anteilen von Vinylpyrrolidonmonomer und einem Ammoniumderivatmonomer abgeleitet ist.

2. Die Haarkonditionierungszusammensetzung nach Anspruch 1, worin das Ammoniumderivatmonomer ein Dialkylaminoalkylacrylat oder-methacrylat ist.

3. Die Haarkonditionierungszusammensetzung nach Anspruch 2, worin das Ammoniumderivatmonomer Dimethylaminoäthylmethacrylat ist.

4. Die Haarkonditionierungszusammensetzung nach Anspruch 3, worin der Mengenanteil an Dimethylaminoäthylmethacrylat 5—10 Gew.-% des Polymeren beträgt.

5. Die Haarkonditionierungszusammensetzung nach einem der vorhergehenden Ansprüche, worin der Mengenanteil an Vinylcaprolactam wenigstens 65 Gew.-% des Terpolymeren und der Mengenanteil an Vinylpyrrolidon nicht mehr als 25 Gew.-% des Terpolymeren beträgt.

6. Die Haarkonditionierungszusammensetzung nach einem der vorhergehenden Ansprüche, worin das Terpolymere auch eine kleinere Menge an Stearylmethacrylat enthält.

7. Die Haarkonditionierungszusammensetzung nach Anspruch 3, worin das Gewichtsverhältnis von Vinylcaprolactam zu Vinylpyrrolidon zu Dimethylaminoäthylmethacrylat ein Verhältnis von etwa 70:25:5 ist.

8. Die Haarkonditionierungszusammensetzung nach Anspruch 7, worin etwa 3 Gew.-% des Vinylcaprolactams durch Stearylmethacrylat ersetzt sind.

9. Die Haarkonditionierungszusammensetzung nach einem der vorhergehenden Ansprüche, worin die Zusammensetzung ein oberflächenaktives Mittel enthält.

**Patentansprüche: AT**

1. Ein Verfahren zur Konditionierung von Haar, umfassend die Anwendung einer Zusammensetzung auf das Haar, die aus einem Termpolymeren besteht oder ein solches enthält, das aus einem größeren Anteil von Vinylcaprolactammonomer und kleineren Anteilen von Vinylpyrrolidonmonomer und einem Ammoniumderivatmonomer abgeleitet ist.

2. Ein Verfahren nach Anspruch 1, worin das Ammoniumderivatmonomer ein Dialkylaminoalkylacrylat oder -methacrylat ist.

3. Ein Verfahren nach Anspruch 2, worin das Ammoniumderivatmonomer Dimethylaminoäthylmethacrylat ist.

4. Ein Verfahren nach Anspruch 3, worin der Mengenanteil an Dimethylaminoäthylmethacrylat 5—10 Gew.-% des Polymeren beträgt.

5. Ein Verfahren nach einem der vorhergehenden Ansprüche, worin der Mengenanteil an Vinylcaprolactam wenigstens 65 Gew.-% des Terpolymeren und der Mengenanteil an Vinylpyrrolidon nicht mehr als 25 Gew.-% des Terpolymeren beträgt.

6. Ein Verfahren nach einem der vorhergehenden Ansprüche, worin das Terpolymere auch eine kleinere Menge an Stearylmethacrylat enthält.

7. Ein Verfahren nach Anspruch 3, worin das Gewichtsverhältnis von Vinylcaprolactam zu Vinylpyrrolidon zu Dimethylaminoäthylmethacrylat ein Verhältnis von etwa 70:25:5 ist.

8. Ein Verfahren nach Anspruch 7, worin etwa 3 Gew.-% des Vinylcaprolactams durch Stearylmethacrylat ersetzt sind.

9. Ein Verfahren nach einem der vorhergehenden Ansprüche, worin die Zusammensetzung ein oberflächenaktives Mittel enthält.

10. Eine Haarkonditionierungszusammensetzung enthaltend ein Terpolymer nach einem der Ansprüche 1—8 und wenigstens ein weiteres Ingrediens.

**Revendications pour les Etats Contractants: BE—CH/LI—DE—FR—GB—IT—NL—SE**

1. Composition de traitement des cheveux constituée d'un terpolymère, ou contenant celui-ci, dérivé

d'une grande proportion d'un monomère de vinylcaprolactame et de petites proportions d'un monomère de vinylpyrrolidone et d'un monomère dérivé d'ammonium.

2. Composition de traitement des cheveux selon la revendication 1, dans laquelle le monomère dérivé d'ammonium est un acrylate ou un méthacrylate de dialkylaminoalkyle.

3. Composition de traitement des cheveux selon la revendication 2, dans laquelle le monomère dérivé d'ammonium est du méthacrylate de diméthylaminoéthyle.

4. Composition de traitement des cheveux selon la revendication 3, dans laquelle la proportion de méthacrylate de diméthylaminoéthyle est de 5 à 10% en poids du polymère.

5. Composition de traitement des cheveux selon l'une quelconque des revendications précédentes, dans laquelle la proportion de vinylcaprolactame est au moins de 65% en poids du terpolymère et la proportion de vinylpyrrolidone ne dépasse pas 25% en poids du terpolymère.

6. Composition de traitement des cheveux selon l'une quelconque des revendications précédentes, dans laquelle le terpolymère contient également une petite quantité de méthacrylate de stéaryle.

7. Composition de traitement des cheveux selon la revendication 3, dans laquelle le rapport pondéral des vinylcaprolactame-vinylpyrrolidone-méthacrylate de diméthylaminoéthyle est d'environ 70/25/5.

8. Composition de traitement des cheveux selon la revendication 7, dans laquelle environ 3% en poids du vinylcaprolactame sont remplacés par du méthacrylate de stéaryle.

9. Composition de traitement des cheveux selon l'une quelconque des revendications précédentes, dans laquelle la composition contient un tensio-actif.

**Revendications pour l'Etat Contractant: Autriche**

1. Procédé de traitement des cheveux comprenant l'application aux cheveux d'une composition constituée d'un terpolymère, ou le contenant, dérivé d'une grande proportion d'un monomère de vinylcaprolactame et de petites proportions d'un monomère de vinylpyrrolidone et d'un monomère dérivé d'ammonium.

2. Procédé selon la revendication 1, dans lequel le monomère dérivé d'ammonium est un acrylate ou méthacrylate de dialkylaminoalkyle.

3. Procédé selon la revendication 2, dans lequel le monomère dérivé d'ammonium est du méthacrylate de diméthylaminoéthyle.

4. Procédé selon la revendication 3, dans lequel la proportion de méthacrylate de diméthylaminoéthyle est de 5 à 10% en poids du polymère.

5. Procédé selon l'une quelconque des revendications précédentes, dans lequel la proportion de vinylcaprolactame est au moins de 65% en poids du terpolymère et la proportion de vinylpyrrolidone ne dépasse pas 25% en poids du terpolymère.

6. Procédé selon l'une quelconque des revendications précédentes, dans lequel le terpolymère contient également une petite quantité de méthacrylate de stéaryle.

7. Procédé selon la revendication 3, dans lequel le rapport pondéral des vinylcaprolactame-vinylpyrrolidone-méthacrylate de diméthylaminoéthyle est d'environ 70/25/5.

8. Procédé selon la revendication 7, dans lequel environ 3% en poids du vinylcaprolactame sont remplacés par du méthacrylate de stéaryle.

9. Procédé selon l'une quelconque des revendications précédentes, dans lequel la composition contient un tensio-actif.

10. Composition de traitement des cheveux contenant un terpolymère selon l'une quelconque des revendications 1 à 8, et au moins un autre composant.

FIGURE 1

EFFECT OF VINYLCAPROLACTAM CONTENT ON
COPOLYMER CLOUD TEMPERATURE